# EUROPEAN PATENT SPECIFICATION

(11) **EP 3 930 792 B1**
(45) Date of publication and mention of the grant of the patent: **23.04.2025**
(21) Application number: 20706285.2
(22) Date of filing: 26.02.2020
(51) Int. Cl.: A61M 5/20, A61M 5/32

(54) **RIGID NEEDLE SHIELD REMOVER FOR AUTOINJECTOR**
NADELSCHUTZENTFERNER FÜR AUTOINJEKTOR
DISPOSITIF DE RETRAIT DE PROTECTION D'AIGUILLE POUR AUTO-INJECTEUR

(30) Priority: 26.02.2019 EP 19305228
(43) Date of publication of application: 05.01.2022
(73) Proprietor: Becton Dickinson France, 38800 Le Pont-de-Claix (FR)
(72) Inventor: PETIT, Louis, 38610 Venon (FR)
(74) Representative: Germain Maureau
(86) International application number: PCT/EP2020/055012
(87) International publication number: WO 2020/173998

(56) References cited:
- WO-A1-2012/103140
- WO-A1-2013/058697
- WO-A1-2016/055334
- WO-A1-2017/223354
- WO-A1-2018/069032

## Description

### BACKGROUND OF THE INVENTION

### Field of the Invention

The present disclosure relates generally to autoinjectors and methods of assembling the same and, more specifically, to devices for removing needle shields from autoinjectors and methods for assembling autoinjectors including such devices.

### Description of Related Art

Automatic drug delivery devices, such as autoinjectors, are designed with removable shields to maintain sterility of the needle and to prevent inadvertent contact between the needle and a user. Many of these needle shields are elastomeric, and ensuring accurate placement and a firm grip between the needle shield and caps or other removal devices can be difficult, resulting in a need for excess force to remove the shield or slippage and an inability to remove the shield at all. Accordingly, a need exists in the art for a needle shield remover that can reliably be positioned for quick and easy removal of the needle shield when the autoinjector is to be used. Related art includes documents WO2013058697A1, WO2016055334A1, WO2018069032A1, WO2017223354A1 and WO2012103140A1.

### SUMMARY OF THE INVENTION

Provided herein is a shield removal assembly for an autoinjector including a syringe barrel, a needle, and a needle shield at least partially surrounding the needle. The shield removal assembly is as defined in claim 1 to 4.

Also provided herein is an autoinjector as defined in claims 6 and 7.

Also provided herein is a method of assembling an autoinjector as defined in claims 8-15.

Further embodiments and aspects are set forth below:

The autoinjector as defined in the claims, wherein the cap remover at least partially surrounds the open distal end of the housing.

The method as defined in the claims, wherein the retainer comprises at least one radially-extending protrusion.

The method described above, wherein the cap remover comprises a shoulder in a sidewall thereof between the proximal end and the distal end of the cap remover.

The autoinjector as defined in the claims, wherein the retainer and the cap remover are arranged to provide lag between initiation of longitudinal movement of the cap remover and initiation of longitudinal movement of the retainer.

The autoinjector described above, wherein the proximal end of the retainer comprises at least one grasping feature.

The autoinjector described above, wherein, in the first position, the proximal end of the needle shield is longitudinally spaced from the at least one grasping feature and in the second position the proximal end of the needle shield abuts the at least one grasping feature.

The autoinjector described above, wherein the retainer comprises at least one member extending proximally from the distal end of the retainer, the at least one member comprising a radially-extending protrusion.

The autoinjector described above, wherein the cap remover is rotatably fixed relative to the housing.

A method of assembling an autoinjector as defined in the claims, wherein the step of inserting a syringe comprises moving the syringe towards the distal end of the lower housing along the longitudinal axis such that: the proximal end of the retainer contacts the syringe barrel proximal to the distal end of the syringe barrel; and the distal end of the needle shield abuts the distal end of the retainer.

The method described above, wherein the retainer comprises at least one member extending proximally from the distal end of the retainer, the at least one member comprising a radially-extending protrusion.

The method described above, wherein the cap remover comprises an opening in a sidewall thereof between the proximal end and the distal end of the cap remover.

A shield removal assembly for an autoinjector as defined in the claims, wherein the proximal end of the retainer is configured to engage the needle shield between the proximal end of the needle shield and the distal end of the syringe barrel, and the distal end comprising an opening therethrough and configured to abut the distal end of the cap remover.

The shield removal assembly described above, wherein the retainer and the cap remover are arranged to provide lag between initiation of longitudinal movement of the cap remover and initiation of longitudinal movement of the retainer.

The shield removal assembly described above, wherein the retainer comprises at least one member extending proximally from the distal end of the retainer, the at least one member comprising a radially-extending protrusion.

The shield removal assembly described above, wherein the lag-providing arrangement defines a longitudinal distance between the radially-extending protrusion of the retainer and a shoulder of the cap remover, such that the cap remover is moved longitudinally away from the syringe a set distance before the radially extending protrusion of the retainer engages the shoulder of the cap remover.

The shield removal assembly described above, wherein the proximal end of the retainer comprises at least one grasping feature.

The shield removal assembly described above, wherein, in the first position, the proximal end of the needle shield is longitudinally spaced from the at least one grasping feature and, in the second position, the proximal end of the needle shield abuts the at least one grasping feature.

### BRIEF DESCRIPTION OF THE DRAWINGS

FIG. 1A is a side view of a shield removal assembly according to a non-limiting embodiment or aspect described herein;
FIG. 1B is a bottom view of a shield removal assembly according to a non-limiting embodiment or aspect described herein;
FIG. 2A is an exploded view of a shield removal assembly according to a non-limiting embodiment or aspect described herein;
FIG. 2B is a perspective view of the shield removal assembly as shown in FIG. 2A;
FIG. 3 is a cross-sectional view of a shield removal assembly according to a non-limiting embodiment or aspect described herein;
FIG. 4A is a perspective view of a shield removal assembly according to a non-limiting embodiment or aspect described herein;
FIG. 4B is a perspective view of a shield removal assembly according to a non-limiting embodiment or aspect described herein;
FIG. 5A is an exploded perspective view of a shield removal assembly according to a non-limiting embodiment or aspect described herein;
FIG. 5B is a perspective view of a cap remover of a shield removal assembly according to a non-limiting embodiment or aspect described herein;
FIG. 5C is a perspective view of a retainer of a shield removal assembly according to a non-limiting embodiment or aspect described herein;
FIG. 6 is a cross-sectional perspective view of a shield removal assembly according to a non-limiting embodiment or aspect described herein;
FIG. 7 is a cross-sectional view of an autoinjector including a shield removal assembly according to a non-limiting embodiment or aspect described herein;
FIG. 8 is a cross-sectional view of an autoinjector including a shield removal assembly according to a non-limiting embodiment or aspect described herein; and
FIG. 9 is a cross-sectional view of an autoinjector including a shield removal assembly according to a non-limiting embodiment or aspect described herein.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

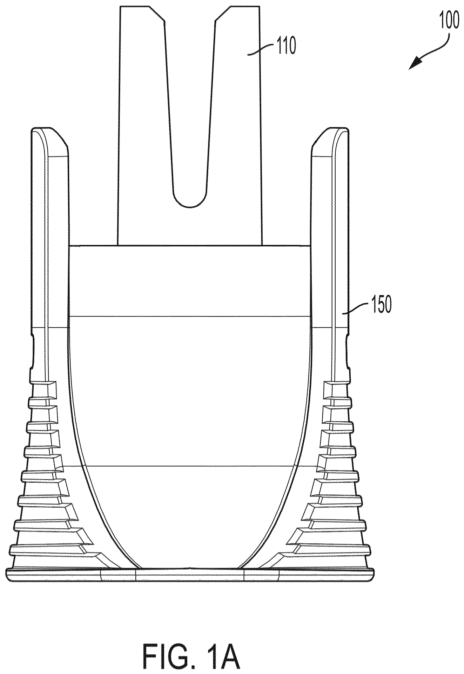

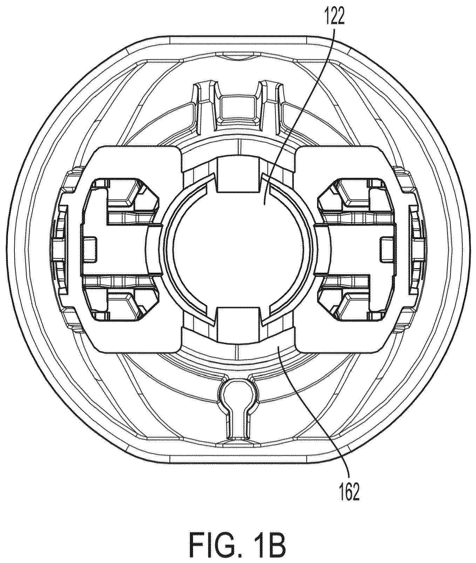

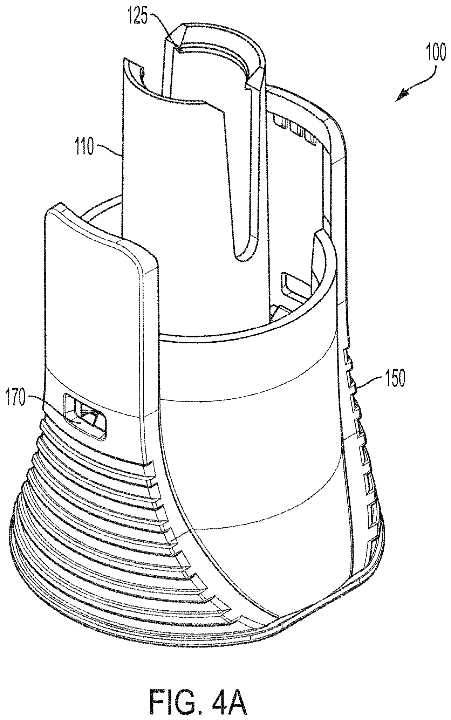

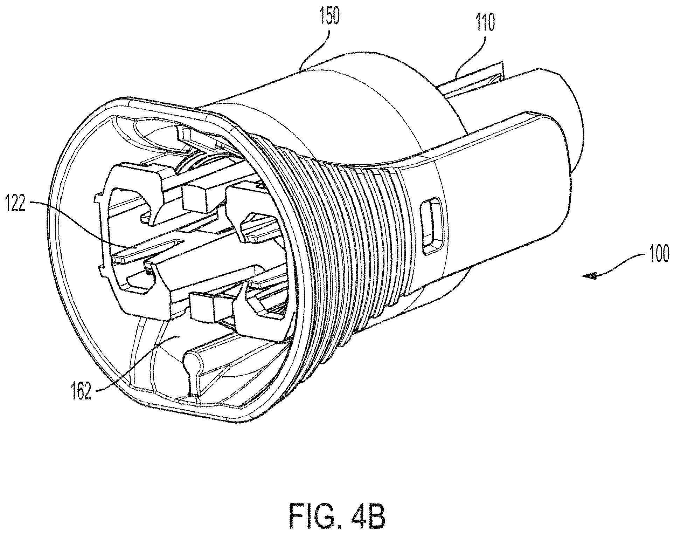

Provided herein is a shield removal assembly for a drug delivery device including a needle. In non-limiting embodiments or aspects, the drug delivery device is an autoinjector or other similar device for automatic or manual delivery of a substance to a user. With reference to **FIGS. 1-6****,** shown is a shield removal assembly (100) including a retainer (110) and a cap remover (150). Retainer (110) is nested within cap remover (150). In non-limiting embodiments or aspects, cap remover (150) is flared at a distal end thereof to form an ergonomic gripping surface to allow a user to firmly grip the cap remover. Retainer (110) and cap remover (150) can be formed of any rigid material, for example, and without limitation, plastics and the like.

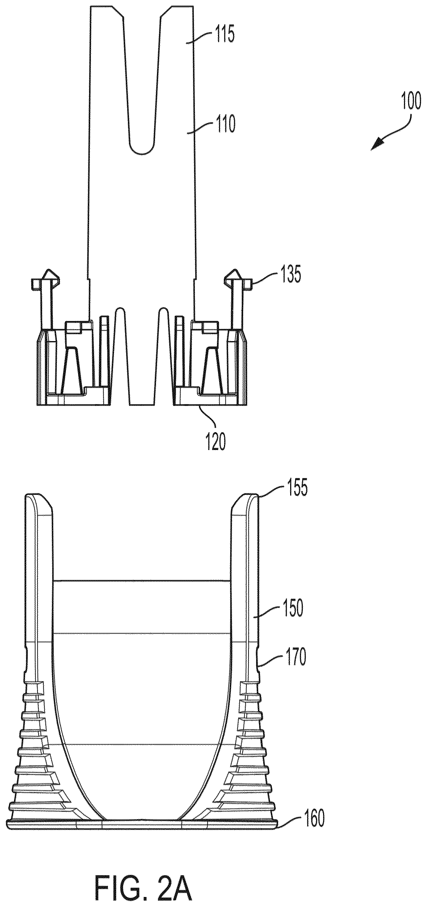

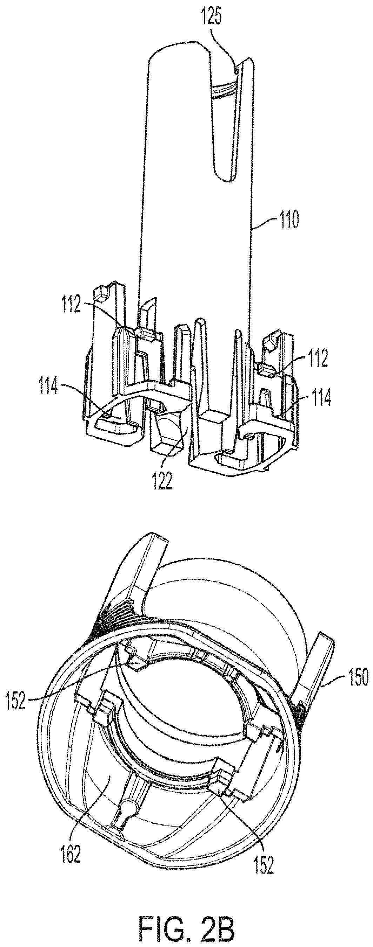

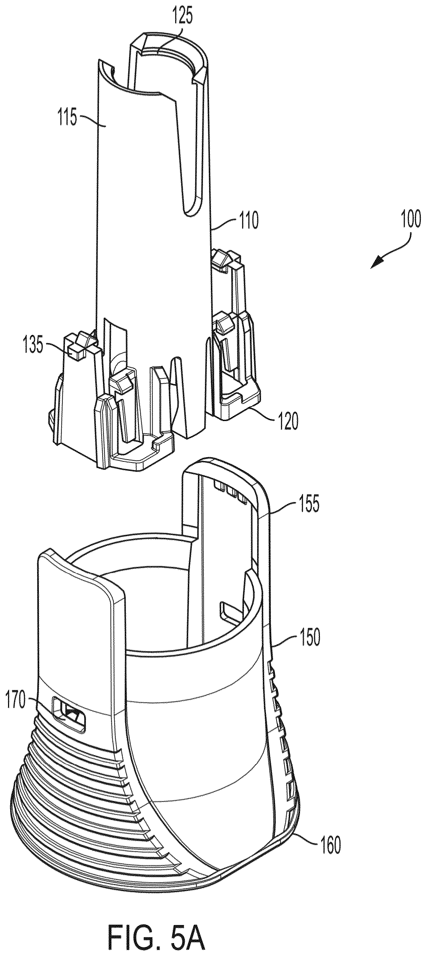

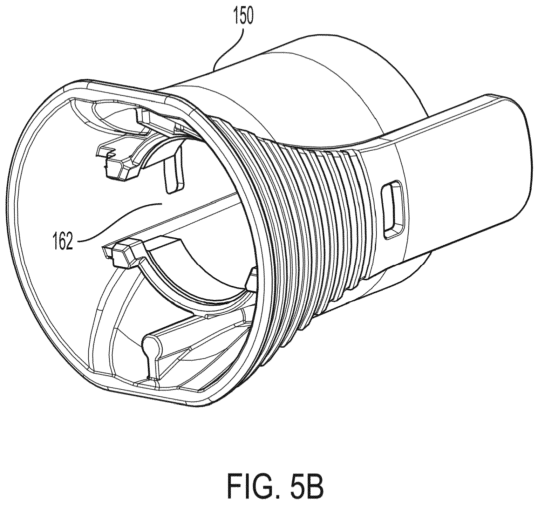

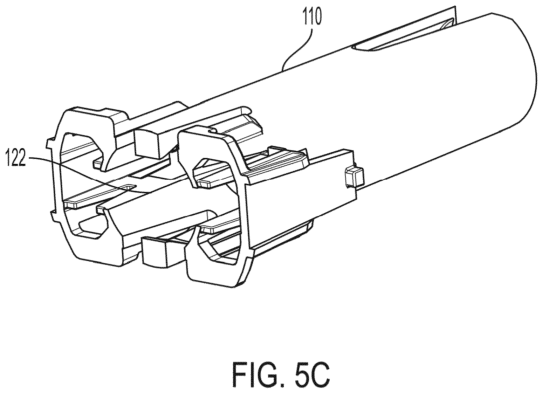

**FIGS. 2A****,** **2B****, and** **5A****-5C** show exploded views of a non-limiting embodiment or aspect of the shield removal assembly described herein. Cap remover (150) includes proximal end (155), which is configured to be closest to the housing, and a distal end (160). In non-limiting embodiments or aspects, proximal end (155) of cap remover (150) includes two or more arms that engage with a housing (not shown) of an autoinjector. In such non-limiting embodiments or aspects, the arms of the cap remover (150) include at least one projection or shoulder to allow secure, releasable engagement with the housing of the autoinjector. The distal end (160) of cap remover (150) includes at least one opening (162) therethrough. In non-limiting embodiments or aspects, cap remover (150) includes in a sidewall thereof at least one opening (170). In non-limiting embodiments or aspects, cap remover (150) includes two openings (170). In non-limiting embodiments or aspects, cap remover (150) includes two openings (170) that are located on opposite sides of the cap remover (150).

With continued reference to **FIGS. 1****,** **2A****,** **2B****,** **4****,** **5A****, and** **5B****,** retainer (110) includes proximal (115, closest to the housing) and distal (120) ends. In non-limiting embodiments or aspects, proximal end (115) of retainer (110) includes two or more arms that engage with the needle shield (not shown) of an autoinjector. In such non-limiting embodiments or aspects, the arms of the retainer (110) include at least one projection to allow secure engagement with the needle shield. The distal end (120) of retainer (110) includes at least one opening (122) therethrough. In non-limiting embodiments, the distal end (120) of the retainer (110) is configured to abut the distal end (160) of the cap remover (150). In non-limiting embodiments or aspects, the opening (162) in the distal end (160) of the cap remover (150) is of a larger diameter than the opening (122) in the distal end (120) of the retainer (110). In non-limiting embodiments or aspects, this difference in diameter allows the retainer (110) to be introduced into the cap remover (150) through the opening (162) in the distal end (160) of the cap remover (150).

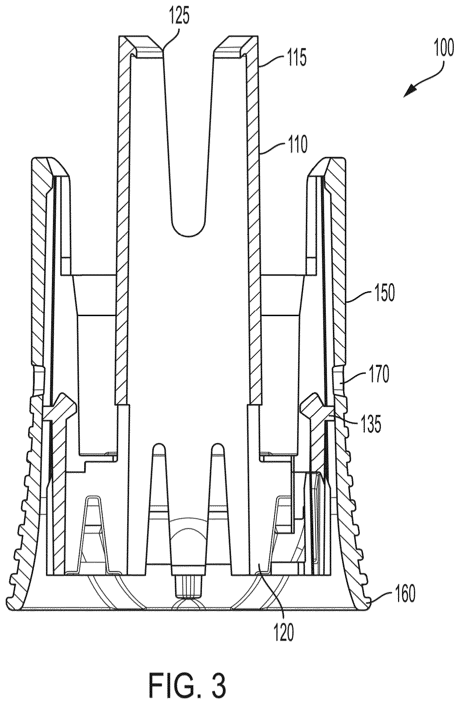

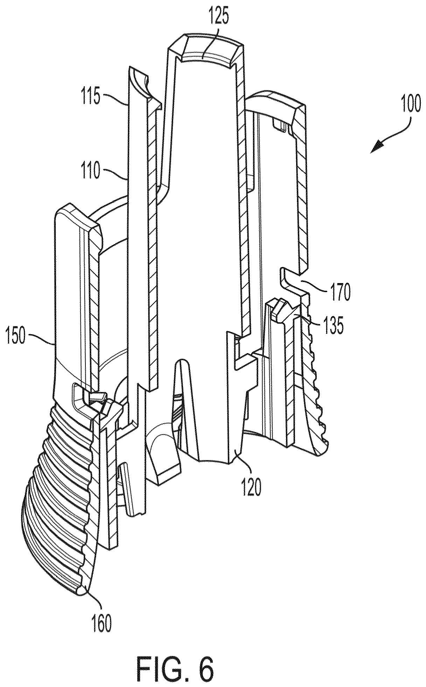

As described previously, proximal end (115) of retainer (110) can include at least one grasping feature, such as a projection, to allow secure engagement with a needle shield. Turning to **FIGS. 3** **and** **6****,** shown is a shield removal assembly according to a non-limiting embodiment or aspect, where the retainer (110) includes at least one grasping feature (125) at a proximal end (115) thereof. The at least one grasping feature can be at least one protrusion (125), and can be configured such that it is capable of gripping a needle shield by a flange thereof.

In non-limiting embodiments or aspects, the retainer (110) and cap remover (150) of the shield removal assembly (100) are configured or arranged such that the needle shield and/or syringe of an autoinjector (not shown) can be shifted relative to the retainer by introducing a device, such as a tool, through the openings (122, 162) in the distal ends (120, 160) of the retainer (110) and cap remover (150), respectively. Placing a tool through the openings in the cap remover and retainer allows the needle shield and syringe to be displaced proximally, and thus allows for adjustment of the positioning of the projections (125) relative to the needle shield to ensure a secure grip between the retainer (110) and the needle shield.

With continued reference to **FIGS. 3** **and** **6****,** as described above in non-limiting embodiments or aspects the cap remover (150) includes at least one opening (170) in a sidewall thereof. The at least one opening (170) is configured to allow at least one radially-extending protrusion (135) on retainer (110) to extend therethrough. In the non-limiting embodiment or aspect shown in **FIGS. 3** **and** **6****,** at least one radially-extending protrusion (135) extends from an arm that extends proximally from the distal end (120) of the retainer (110). In other non-limiting embodiments or aspects (not shown), the at least one radially-extending protrusion (135) is positioned on a main body of the retainer (110).

With reference to **FIGS. 2A** **and** **2B****,** a lag arrangement is provided between the cap remover (150) and the retainer (110) during removal of the shield removal assembly (100). Retainer (110) is received within cap remover (150) such that at least one protrusion (112) is arranged proximally of at least one shoulder (152) of cap remover (150). In non-limiting embodiments or aspects, the at least one protrusion (112) prevents the retainer (110) from being displaced distally out of the cap remover (150), by virtue of engagement with a proximal surface of the at least one shoulder (152). In non-limiting embodiments or aspects, the retainer (110) further includes at least one flange (114), arranged distally of the at least one shoulder (152) of the cap remover (150). In this way, the retainer (110) is prevented from being displaced proximally out of the cap remover (150) by virtue of engagement of a proximal surface of the at least one flange (114) with a distal surface of the at least one shoulder (152). The longitudinal space between the at least one protrusion (112) and the at least one flange (114) on the retainer (110) results in a predetermined amount of "play" between these components. Accordingly, in non-limiting embodiments or aspects, when the cap remover (150) is pulled distally to remove the shield removal assembly (100) from a drug delivery device to which it is attached, cap remover (150) is pulled a certain distance before the at least one shoulder (152) engages the at least one protrusion (112) of the retainer (110). This results in a lag between the initiation of movement of the cap remover (150) and initiation of movement of the retainer (110). When cap remover (150) moves sufficiently such that the at least one shoulder (152) engages with the at least one protrusion (112), the retainer (110) begins to be pulled away from the drug delivery device, and, because of the positioning of the at least one projection (125) relative to the needle shield, the needle shield begins to be pulled away from the syringe and needle.

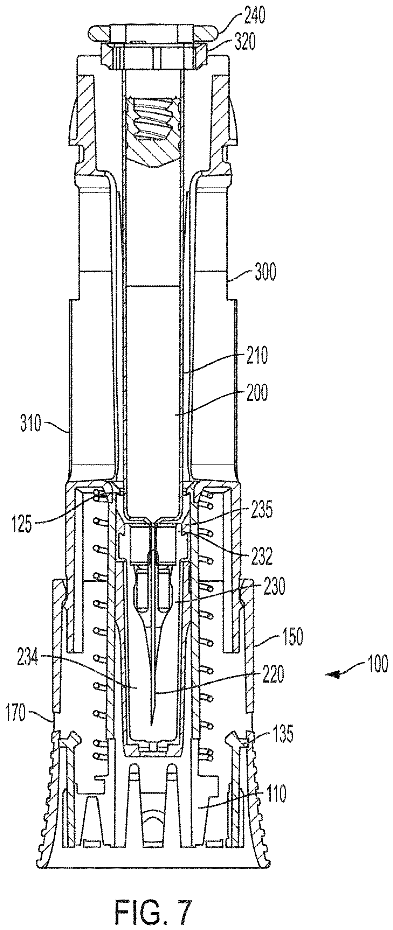

Also provided herein is an autoinjector including a housing, a syringe having a barrel, a needle, and a needle shield, and a shield removal assembly. Turning to **FIGS. 7-9****,** shown is an autoinjector having a housing, a syringe (200) having a barrel (210) and a needle (220) attached thereto, a removable needle shield (230) configured to at least partially surround the needle (220), and a shield removal assembly (100). The autoinjector housing (a lower housing (310) is shown, though it should be understood that autoinjector can include an upper housing, as described below) includes a proximal end and a distal end, where the distal end is open to permit the needle (220) to pass therethrough to allow a substance to be delivered to a user.

Removable needle shield (230) can include proximal (232) and distal (234) ends. In non-limiting embodiments or aspects, removable needle shield (230) is a two-piece needle shield, including an elastomeric inner portion and a rigid outer portion. In non-limiting embodiments or aspects, the rigid outer portion at least partially surrounds the elastomeric inner portion of the needle shield (230). In non-limiting embodiments or aspects, needle shield (230) includes at least one flange or shoulder (235). In non-limiting embodiments or aspects, the at least one flange or shoulder (235) is located on the elastomeric inner portion, for engaging the rigid outer portion. In non-limiting embodiments or aspects, needle shield (230) includes at least one complementary grasping feature (233) on the proximal end (232) thereof. In non-limiting embodiments or aspects, the at least one complementary grasping feature (233) is configured to be complementary to, and engageable with, the at least one grasping feature (125) of the retainer. In non-limiting embodiments or aspects, the at least one complementary grasping feature (233) is the proximal edge of the needle shield (230).

In non-limiting embodiments or aspects, the elastomeric portion of the needle shield (230) is formed of one or more natural or synthetic rubbers and/or of thermoplastic elastomer, or combinations thereof. In non-limiting embodiments or aspects, the rigid outer portion of the needle shield (230) is formed of a rigid plastic. In non-limiting embodiments or aspects, the rigid outer portion is formed of polypropylene.

Shield removal assembly (100) includes, as described previously, cap remover (150) and retainer (110). In non-limiting embodiments or aspects, the cap remover (150) and/or retainer (110) include one or more of the features described above. In non-limiting embodiments or aspects, cap remover (150) at least partially surrounds the open distal end of the housing. In non-limiting embodiments or aspects, cap remover (150) is not rotatable relative to the housing. Autoinjector housing and/or cap remover (150) can include suitable features to prevent rotation of the cap remover (150), and, accordingly, retainer (110) and needle shield (230), relative to the housing and the needle (220), during needle shield (230) removal. In non-limiting embodiments or aspects, suitable anti-rotation features include one or more projections, guide channels, and/or shoulders on cap remover (150) and/or housing.

Also provided herein is a method of assembling an autoinjector including a shield removal assembly, including the steps of providing a lower housing assembly of an autoinjector, inserting a syringe into the lower assembly, and placing an upper assembly on the lower assembly. In a first step, a lower housing assembly (300), including shield removal assembly (100) engaged therewith, is provided. The lower housing assembly (300) includes lower housing (310). In non-limiting embodiments or aspects, lower assembly (300) includes a ring (320) at a proximal end thereof. The lower housing assembly (300) includes a proximal end and a distal end, the distal end being open to allow a needle of a syringe to pass therethrough, and the proximal end of the lower housing (310) being open to allow a syringe to be inserted into the housing. Housing may be formed of any suitable material, including, for example and without limitation, a rigid plastic. Lower assembly (300) also includes a shield removal assembly (100). Shield removal assembly (100) includes, as described previously, cap remover (150) and retainer (110), each having distal ends (160, 120) having an opening (162, 122) therethrough. In non-limiting embodiments or aspects, the cap remover (150) and/or retainer (110) include one or more of the features described above. In an initial configuration (e.g., when shield removal assembly (100) is positioned on lower housing (300)), the distal end (120) of the retainer (110) is spaced from the distal end (160) of the cap remover (150).

With reference to **FIG. 7****,** in non-limiting embodiments or aspects, in a second step of the method, a syringe (200) is inserted into the lower housing (310). The syringe (200) can include a barrel (210) to hold a medicament or other substance therein, a needle (220) arranged at a distal end of the barrel (210), and a removable needle shield (230). Syringe (200), barrel (210), needle (220), and needle shield (230) can include one or more of the features described above. Syringe barrel (210) can be of any useful size, and can be formed of any useful material, such as glass or plastic. In non-limiting embodiments or aspects, syringe (200) is a unitary syringe, and the needle (220) is formed with the syringe barrel (210). In non-limiting embodiments or aspects, syringe barrel (210) is not formed with needle (220) therein and, instead, a needle hub (not shown) holds needle (220). Syringe needle (220) can be formed of any useful material, and can be of any suitable gauge. Needle shield (230) can be a one-piece needle shield formed of an elastomeric material, or a two-piece needle shield having an inner portion formed of an elastomeric material and an outer portion formed of a rigid material. The needle shield (230) at least partially surrounds the needle (220). In non-limiting embodiments or aspects, syringe (200) includes a flange (240) at a proximal end thereof. At the conclusion of the second step, the distal end (120) of the retainer (110) remains longitudinally spaced from the distal end (160) of the cap remover (150).

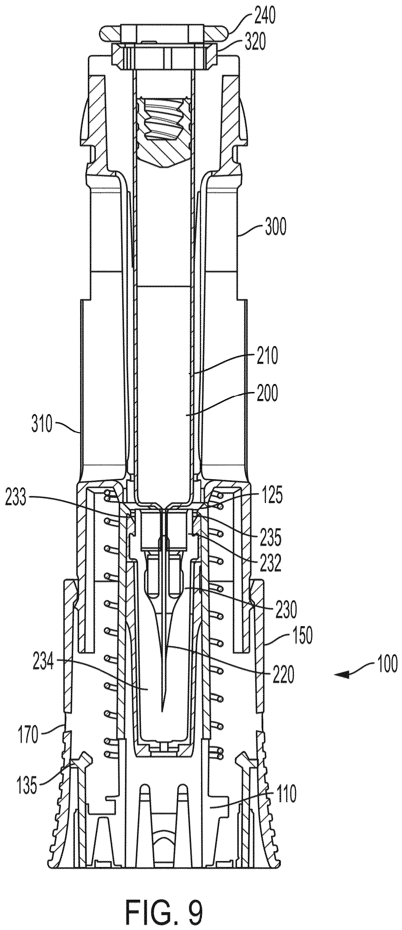

In non-limiting embodiments or aspects, the step of inserting the syringe (200) includes moving the syringe (200) towards the distal end of the housing (310), preferably such that the at least one grasping feature (125) is spaced longitudinally from the proximal end of the needle shield. In other words, the at least one grasping feature (125) is more proximal than in its final position (shown in **FIG. 9**). In this position, the distal end (234) of the syringe flange abuts the ring (320). In this position, the proximal end (115) of the retainer (110) contacts the syringe barrel (210).

As described above, at the conclusion of this step, the distal end (120) of the retainer (110) is maintained proximally with respect to the distal end (160) of the cap remover (150), such that the distal end (120) of the retainer (110) is longitudinally spaced from the distal end (160) of the cap remover (150). In non-limiting embodiments or aspects, a tool (not shown) is inserted within the opening (162) of the cap remover (150). This tool can be used to push the retainer (110) proximally, such that the distal end (120) of the retainer (110) is longitudinally spaced from the distal end (160) of the cap remover (150).

In non-limiting embodiments or aspects, the proximal end (115) of the retainer (110) includes at least one grasping feature, such as projections (125), and the projections (125) contact the syringe barrel (210) proximally of the distal end of the syringe barrel (210). In this way, the retainer (110) is in an "overstroked" position, where the at least one grasping feature (125) is located too far proximally of the needle shield (230) **(****FIG. 7****).** In non-limiting embodiments or aspects, the needle shield (230) includes at least one flange or shoulder (235), and the proximal end (115) of the retainer (110) includes at least one grasping feature (125), and the at least one grasping feature (125) contacts the syringe barrel (210) proximally of the at least one flange or shoulder (235).

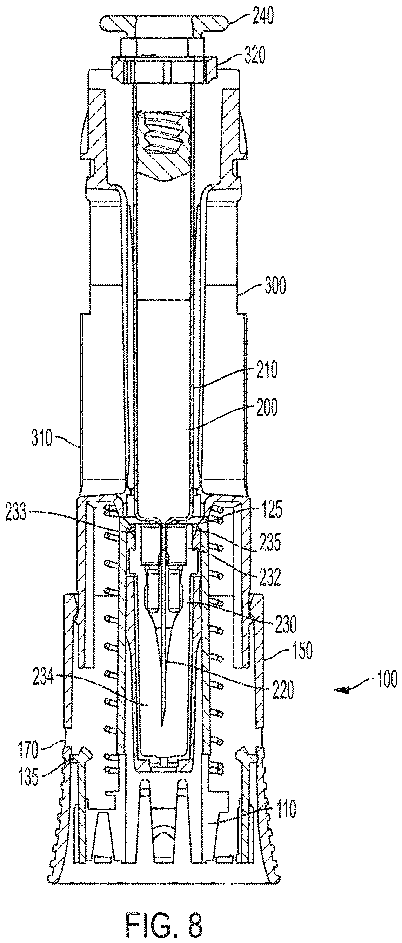

With reference to **FIG. 8****,** in non-limiting embodiments or aspects, a third step of the method of assembling the syringe includes, after inserting the syringe (200) into the lower housing (310), moving the syringe (200) proximally. In non-limiting embodiments or aspects, moving the syringe (200) proximally within the lower housing (310) is achieved by inserting a device through the at least one opening (162, 122) in the distal ends (160, 120) of the cap remover (150) and retainer (110), and applying pressure to urge the syringe (200) proximally. In non-limiting embodiments or aspects, and as shown in **FIG. 8****,** when the syringe (200) is urged proximally, the at least one grasping feature (125) of the retainer (110) engages the needle shield at a proximal-most end thereof. In non-limiting embodiments or aspects, the at least one grasping feature (125) engages at least one corresponding, complementary grasping feature (233) on the needle shield (230). In non-limiting embodiments or aspects, after the syringe (200) is urged proximally, the syringe flange (240) is longitudinally spaced from the ring (320) of the lower assembly (300). At the end of this step the at least one grasping feature (125) engages the at least one complementary grasping feature (233) of the needle shield (230), the distal end (120) of the retainer (110) is longitudinally spaced from the distal end (160) of the cap remover (150), and the syringe flange (240) is longitudinally spaced from the ring (320).

In non-limiting embodiments or aspects, the method includes a fourth step of placing an upper assembly (not shown), including an upper housing, on the lower assembly. As with lower assembly and lower housing, upper assembly and upper housing can be formed of any suitable material, including, for example and without limitation, a rigid plastic. In non-limiting embodiments or aspects, the step of placing the upper assembly on the lower assembly causes the syringe to move distally, such that the syringe flange (240) abuts the ring (320).

With reference to **FIG. 9****,** after assembly, distal end (120) of retainer (110) falls or is otherwise displaced towards the distal end (160) of the cap remover (150), such that the distal end (120) of the retainer (110) abuts the distal end (160) of the cap remover (150).

Once a final user wants to remove the needle shield, he/she pulls the cap remover. As described above, the gap between the at least one protrusion (112) of the retainer (110) and the at least one shoulder (152) of the cap remover (150) causes a lag between the initiation of longitudinal movement of the cap remover (150) away from the lower housing assembly (300) and the initiation of longitudinal movement of the retainer (110) away from the lower housing assembly (300).

Although the invention has been described in detail for the purpose of illustration based on what is currently considered to be the most practical and preferred embodiments, it is to be understood that such detail is solely for that purpose and that the invention is not limited to the disclosed embodiments, but on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

## Claims

1. A shield removal assembly (100) for an autoinjector comprising a syringe barrel, a needle, and a needle shield at least partially surrounding the needle, the shield removal assembly comprising:
a cap remover (150) configured to be grasped by a user and having a proximal end (155) and a distal end (160), the distal end (160) of the cap remover (150) comprising at least one opening (162) therethrough; and
a retainer (110) received within the cap remover (150) and having a proximal end (115) and a distal end (120), the retainer (110) comprising at least one grasping feature (125) configured to engage at least one complementary grasping feature of the needle shield, and the distal end (120) comprising an opening (122) therethrough;
wherein the shield removal assembly (100) is configured such that the needle shield of the autoinjector can be advanced through the shield removal assembly (100) from a first position in which the at least one grasping feature (125) is spaced longitudinally and located proximally of the at least one complementary grasping feature of the needle shield to a second position in which the at least one grasping feature (125) engages the at least one complementary grasping feature of the needle shield;
wherein the retainer (110) comprises at least one radially-extending protrusion (135), and the cap remover (150) comprises at least one window (170) configured to receive the radially-extending protrusion (135), and
**characterized in that** the retainer is axially moveable with respect to the cap remover,
and **in that** the retainer comprises at least one protrusion (112); and the cap remover (150) comprises at least one shoulder (152) longitudinally spaced from the at least one protrusion (112) of the retainer (110), such that the cap remover (150) is moved longitudinally away from the syringe a set distance before the at least one shoulder (152) engages the at least one protrusion (112).

2. . The shield removal assembly of claim 1, wherein the at least one grasping feature comprises at least one protrusion, and the at least one complementary grasping feature comprises a proximal edge of the needle shield.

3. . The shield removal assembly (100) of any of claims 1-2, wherein the at least one grasping feature (125) is located at the proximal end (115) of the retainer (110), the shield removal assembly (100) configured such that the needle shield of the autoinjector can be advanced through the shield removal assembly (100) from a first position in which the proximal end of the needle shield is longitudinally spaced from the at least one grasping feature (125) of the retainer (110) to a second position in which the proximal end of the needle shield abuts the at least one grasping feature (125) of the retainer (110).

4. . The shield removal assembly (100) of any of claims 1-3, wherein the opening in the distal end (160) of the cap remover (150) has a greater diameter than the opening in the distal end (120) of the retainer (110).

5. . The shield removal assembly (100) of any of claims 1-4, wherein, after assembly, distal end (120) of retainer (110) falls or is otherwise displaced towards the distal end (160) of the cap remover (150), such that the distal end (120) of the retainer (110) abuts the distal end (160) of the cap remover (150).

6. . An autoinjector comprising:
a housing having a proximal end, an open distal end, and defining a longitudinal axis;
a syringe (200) held within the housing and having a barrel (210);
a needle (220) attached to a distal end of the syringe barrel (210);
a removable needle shield (230) having a proximal end (232) and a distal end, the needle shield (230) configured to at least partially surround the needle (220); and
a shield removal assembly (100) according to any of claims 1-5.

7. . The autoinjector of claim 6, wherein the cap remover (150) is not rotatable relative to the housing.

8. . A method of assembling an autoinjector comprising:
providing a lower assembly (300), the lower assembly comprising:
a lower housing (310) having a proximal end and a distal end, and defining a longitudinal axis;
and a shield removal assembly (100), according to any of claims 1-5, arranged at the distal end of the lower housing (310), wherein the at least one opening (122) of the retainer is spaced from the distal end (160) of the cap remover (150) in an initial configuration;
inserting a syringe (200) into the lower assembly, the syringe (200) comprising:
a barrel (210) having a proximal end and a distal end;
a needle (220) arranged at the distal end of the barrel (210); and
a removable needle shield (230) having a proximal end (232) comprising at least one complimentary grasping feature (233) and a distal end (234), the needle shield (230) configured to at least partially surround the needle (220),
wherein the step of inserting comprises moving the syringe (200) towards the distal end of the lower housing (310) along the longitudinal axis such that the at least one grasping feature (125) of the retainer (110) is spaced longitudinally and located proximally of the at least one complementary grasping feature (233) of the needle shield (230); and
placing an upper assembly comprising an upper housing on the lower assembly (300).

9. . The method of claim 8, wherein the at least one grasping feature (125) of the retainer (110) is a projection (125) configured to engage a proximal edge of the needle shield (230), and wherein the step of inserting the syringe (200) into the lower assembly comprises urging the syringe (200) distally such that the at least one projection (125) is positioned proximally and is longitudinally spaced from the proximal edge of the needle shield (230).

10. . The method of claim 8 or claim 9, further comprising a step of, after inserting the syringe (200) into the lower assembly (300), urging the syringe (200) proximally such that the at least one grasping feature (125) engages the proximal edge of the needle shield.

11. . The method of claim 10, wherein the step of urging the syringe (200) proximally comprises inserting a device through the at least one opening (162) in the distal end (160) of the cap remover (150) and the opening (122) in the distal end (120) of the retainer (110) to urge the syringe (200) proximally.

12. . The method of any of claims 9-11, wherein the proximal end of the syringe barrel (210) comprises a flange (240) and the proximal end of the lower housing (310) comprises a ring (320).

13. . The method of claim 12, wherein, after insertion of the syringe (200), the syringe flange (240) abuts the ring (320).

14. . The method of claim 13, further comprising, after inserting the syringe (200) into the lower assembly (300), a step of urging the syringe (200) proximally, and wherein after the step of urging the syringe (200) proximally, the syringe flange (240) is longitudinally spaced from the ring (320) of the needle shield.

15. . The method of any of claims 8-14, wherein after assembly, distal end (120) of retainer (110) falls or is otherwise displaced towards the distal end (160) of the cap remover (150), such that the distal end (120) of the retainer (110) abuts the distal end (160) of the cap remover (150).

## Patentansprüche

1. Schutzentfernungsbaugruppe (100) für einen Autoinjektor, umfassend einen Spritzenkörper, eine Nadel und einen Nadelschutz, der die Nadel mindestens teilweise umgibt, wobei die Schutzentfernungsbaugruppe Folgendes umfasst:
einen Kappenentferner (150), der so eingerichtet ist, dass er von einem Benutzer gegriffen werden kann und ein proximales Ende (155) und ein distales Ende (160) aufweist, wobei das distale Ende (160) des Kappenentferners (150) mindestens eine Öffnung (162) darin umfasst; und
eine Halterung (110), die innerhalb des Kappenentferners (150) aufgenommen ist und ein proximales Ende (115) und ein distales Ende (120) umfasst, wobei die Halterung (110) mindestens ein Greifelement (125) umfasst, das so eingerichtet ist, dass es mindestens ein ergänzendes Greifelement des Nadelschutzes einrastet, und das distale Ende (120) eine Öffnung (122) darin umfasst;
wobei die Schutzentfernungsbaugruppe (100) so eingerichtet ist, dass der Nadelschutz des Autoinjektors durch die Schutzentfernungsbaugruppe (100) von einer ersten Position vorgeschoben werden kann, in der das mindestens eine Greifelement (125) längs beabstandet ist und sich proximal des mindestens einen ergänzenden Greifelements des Nadelschutzes befindet, bis zu einer zweiten Position, in der das mindestens eine Greifelement (125) das mindestens eine ergänzende Greifelement des Nadelschutzes einrastet;
wobei die Halterung (110) mindestens einen radial erstreckbaren Vorsprung (135) umfasst, und der Kappenentferner (150) mindestens ein Fenster (170) umfasst, das so eingerichtet ist, dass es den radial erstreckbaren Vorsprung (135) aufnimmt, und
**dadurch gekennzeichnet, dass** die Halterung relativ zum Kappenentferner axial beweglich ist,
und dass die Halterung mindestens einen Vorsprung (112) umfasst; und der Kappenentferner (150) mindestens eine Schulter (152) umfasst, die längs von dem mindestens einen Vorsprung (112) der Halterung (110) beabstandet ist, so dass der Kappenentferner (150) längs um einen bestimmten Abstand von der Spritze wegbewegt wird, bevor die mindestens eine Schulter (152) den mindestens einen Vorsprung (112) einrastet.

2. Schutzentfernungsbaugruppe nach Anspruch 1, wobei das mindestens eine Greifelement mindestens einen Vorsprung umfasst und das mindestens eine ergänzende Greifelement einen proximalen Rand des Nadelschutzes umfasst.

3. Schutzentfernungsbaugruppe (100) nach einem der Ansprüche 1 bis 2, wobei sich das mindestens eine Greifelement (125) am proximalen Ende (115) der Halterung (110) befindet, wobei die Schutzentfernungsbaugruppe (100) so eingerichtet ist, dass der Nadelschutz des Autoinjektors aus einer ersten Position, in der das proximale Ende des Nadelschutzes längs von dem mindestens einen Greifelement (125) der Halterung (110) beabstandet ist, durch die Schutzentfernungsbaugruppe (100) vorgeschoben werden kann in eine zweite Position, in der das proximale Ende des Nadelschutzes an dem mindestens einen Greifelement (125) der Halterung (110) anliegt.

4. Schutzentfernungsbaugruppe (100) nach einem der Ansprüche 1 bis 3, wobei die Öffnung im distalen Ende (160) des Kappenentferners (150) einen größeren Durchmesser hat als die Öffnung im distalen Ende (120) der Halterung (110).

5. Schutzentfernungsbaugruppe (100) nach einem der Ansprüche 1 bis 4, wobei nach dem Zusammenbau das distale Ende (120) der Halterung (110) in Richtung des distalen Endes (160) des Kappenentferners (150) fällt oder anderweitig verschoben wird, so dass das distale Ende (120) der Halterung (110) am distalen Ende (160) des Kappenentferners (150) anliegt.

6. Autoinjektor, umfassend:
ein Gehäuse, das ein proximales Ende, ein offenes distales Ende aufweist und eine Längsachse definiert;
eine Spritze (200), die innerhalb des Gehäuses gehalten wird und einen Zylinder (210) aufweist;
eine Nadel (220), die an einem distalen Ende des Spritzenkörpers (210) befestigt ist;
einen abnehmbaren Nadelschutz (230), der ein proximales Ende (232) und ein distales Ende aufweist, wobei der Nadelschutz (230) so eingerichtet ist, dass er die Nadel (220) mindestens teilweise umgibt; und
eine Schutzentfernungsbaugruppe (100) nach einem der Ansprüche 1 bis 5.

7. Autoinjektor nach Anspruch 6, wobei der Kappenentferner (150) relativ zum Gehäuse nicht drehbar ist.

8. Verfahren zur Montage eines Autoinjektors, umfassend:
Bereitstellung einer unteren Baugruppe (300), wobei die untere Baugruppe umfasst:
ein unteres Gehäuse (310), das ein proximales Ende und ein distales Ende aufweist und eine Längsachse definiert;
und einer Schutzentfernungsbaugruppe (100) nach einem der Ansprüche 1 bis 5, die am distalen Ende des unteren Gehäuses (310) angeordnet ist, wobei die mindestens eine Öffnung (122) der Halterung in einer anfänglichen Einrichtung von dem distalen Ende (160) des Kappenentferners (150) beabstandet ist;
Einsetzen einer Spritze (200) in die untere Baugruppe, wobei die Spritze (200) Folgendes umfasst:
einen Zylinder (210) mit einem proximalen Ende und einem distalen Ende;
eine Nadel (220), die am distalen Ende des Zylinders (210) angeordnet ist; und
einen abnehmbaren Nadelschutz (230), der ein proximales Ende (232) aufweist, das mindestens ein ergänzendes Greifelement (233) und ein distales Ende (234) umfasst, wobei der Nadelschutz (230) so eingerichtet ist, dass er die Nadel (220) zumindest teilweise umgibt,
wobei der Schritt des Einsetzens das Bewegen der Spritze (200) in Richtung des distalen Endes des unteren Gehäuses (310) entlang der Längsachse umfasst, so dass das mindestens ein Greifelement (125) der Halterung (110) längs beabstandet ist und sich proximal des mindestens einen ergänzenden Greifelements (233) des Nadelschutzes (230) befindet; und
Aufsetzen einer oberen Baugruppe, die ein oberes Gehäuse umfasst, auf die untere Baugruppe (300).

9. Verfahren nach Anspruch 8, wobei das mindestens eine Greifelement (125) der Halterung (110) ein Vorsprung (125) ist, der so eingerichtet ist, dass er einen proximalen Rand des Nadelschutzes (230) einrastet, und wobei der Schritt des Einsetzens der Spritze (200) in die untere Baugruppe das distale Einführen der Spritze (200) umfasst, so dass der mindestens eine Vorsprung (125) proximal positioniert ist und längs vom proximalen Rand des Nadelschutzes (230) beabstandet ist.

10. Verfahren nach Anspruch 8 oder Anspruch 9, ferner umfassend einen Schritt, bei dem nach dem Einsetzen der Spritze (200) in die untere Baugruppe (300) die Spritze (200) proximal so gedrückt wird, dass das mindestens eine Greifelement (125) den proximalen Rand des Nadelschutzes einrastet.

11. Verfahren nach Anspruch 10, wobei der Schritt des proximalen Eindringens der Spritze (200) das Einsetzen einer Vorrichtung durch die mindestens eine Öffnung (162) in das distale Ende (160) des Kappenentferners (150) und die Öffnung (122) in das distale Ende (120) der Halterung (110) umfasst, um die Spritze (200) proximal einzudringen.

12. Verfahren nach einem der Ansprüche 9 bis 11, wobei das proximale Ende des Spritzenkörpers (210) einen Flansch (240) und das proximale Ende des unteren Gehäuses (310) einen Ring (320) umfasst.

13. Verfahren nach Anspruch 12, wobei nach dem Einsetzen der Spritze (200) der Spritzenflansch (240) am Ring (320) anliegt.

14. Verfahren nach Anspruch 13, ferner umfassend, nach dem Einsetzen der Spritze (200) in die untere Baugruppe (300), einen Schritt des proximalen Eindringens der Spritze (200), und wobei nach dem Schritt des proximalen Eindringens der Spritze (200) der Spritzenflansch (240) längs vom Ring (320) des Nadelschutzes beabstandet ist.

15. Verfahren nach einem der Ansprüche 8 bis 14, wobei nach dem Zusammenbau das distale Ende (120) der Halterung (110) in Richtung des distalen Endes (160) des Kappenentferners (150) fällt oder anderweitig verschoben wird, sodass das distale Ende (120) der Halterung (110) am distalen Ende (160) des Kappenentferners (150) anliegt.

## Revendications

1. Ensemble de retrait de protection (100) pour un auto-injecteur comprenant un corps de seringue, une aiguille et une protection d'aiguille entourant au moins partiellement l'aiguille, l'ensemble de retrait de protection comprenant :
un dispositif de retrait de capuchon (150) configuré pour être saisi par un utilisateur et ayant une extrémité proximale (155) et une extrémité distale (160), l'extrémité distale (160) du dispositif de retrait de capuchon (150) comprenant au moins une ouverture (162) à travers celle-ci ; et
un dispositif de retenue (110) reçu à l'intérieur du dispositif de retrait de capuchon (150) et ayant une extrémité proximale (115) et une extrémité distale (120), le dispositif de retenue (110) comprenant au moins un élément de saisie (125) configuré pour venir en prise avec au moins un élément de saisie complémentaire de la protection d'aiguille, et l'extrémité distale (120) comprenant une ouverture (122) à travers celle-ci ;
dans lequel l'ensemble de retrait de protection (100) est configuré de sorte que la protection d'aiguille de l'auto-injecteur puisse être avancée à travers l'ensemble de retrait de protection (100) depuis une première position dans laquelle l'au moins un élément de saisie (125) est espacé longitudinalement de et situé de manière proximale par rapport à l'au moins un élément de saisie complémentaire de la protection d'aiguille jusqu'à une seconde position dans laquelle l'au moins un élément de saisie (125) vient en prise avec l'au moins un élément de saisie complémentaire de la protection d'aiguille ;
dans lequel le dispositif de retenue (110) comprend au moins une protubérance s'étendant radialement (135), et le dispositif de retrait de capuchon (150) comprend au moins une fenêtre (170) configurée pour recevoir la protubérance s'étendant radialement (135), et
**caractérisé en ce que** le dispositif de retenue est mobile axialement par rapport au dispositif de retrait de capuchon,
et **en ce que** le dispositif de retenue comprend au moins une protubérance (112) ; et le dispositif de retrait de capuchon (150) comprend au moins un épaulement (152) espacé longitudinalement de l'au moins une protubérance (112) du dispositif de retenue (110), de sorte que le dispositif de retrait de capuchon (150) soit éloigné longitudinalement de la seringue d'une distance définie avant que l'au moins un épaulement (152) ne vienne en prise avec l'au moins une protubérance (112).

2. Ensemble de retrait de protection de la revendication 1, dans lequel l'au moins un élément de saisie comprend au moins une protubérance, et l'au moins un élément de saisie complémentaire comprend un bord proximal de la protection d'aiguille.

3. Ensemble de retrait de protection (100) de l'une des revendications 1 et 2, dans lequel l'au moins un élément de saisie (125) est situé à l'extrémité proximale (115) du dispositif de retenue (110), l'ensemble de retrait de protection (100) étant configuré de sorte que la protection d'aiguille de l'auto-injecteur puisse être avancée à travers l'ensemble de retrait de protection (100) depuis une première position dans laquelle l'extrémité proximale de la protection d'aiguille est espacée longitudinalement de l'au moins un élément de saisie (125) du dispositif de retenue (110) jusqu'à une seconde position dans laquelle l'extrémité proximale de la protection d'aiguille vient en butée contre l'au moins un élément de saisie (125) du dispositif de retenue (110).

4. Ensemble de retrait de protection (100) de l'une des revendications 1 à 3, dans lequel l'ouverture dans l'extrémité distale (160) du dispositif de retrait de capuchon (150) a un diamètre supérieur à celui de l'ouverture dans l'extrémité distale (120) du dispositif de retenue (110).

5. Ensemble de retrait de protection (100) de l'une des revendications 1 à 4, dans lequel, après l'assemblage, l'extrémité distale (120) du dispositif de retenue (110) tombe ou est déplacée d'une autre manière vers l'extrémité distale (160) du dispositif de retrait de capuchon (150), de sorte que l'extrémité distale (120) du dispositif de retenue (110) vienne en butée contre l'extrémité distale (160) du dispositif de retrait de capuchon (150).

6. Auto-injecteur comprenant :
un carter ayant une extrémité proximale, une extrémité distale ouverte, et définissant un axe longitudinal ;
une seringue (200) maintenue à l'intérieur du carter et ayant un corps (210)
une aiguille (220) fixée à une extrémité distale du corps de seringue (210)
une protection d'aiguille amovible (230) ayant une extrémité proximale (232) et une extrémité distale, la protection d'aiguille (230) étant configurée pour entourer au moins partiellement l'aiguille (220) ; et
un ensemble de retrait de protection (100) selon l'une des revendications 1 à 5.

7. Auto-injecteur de la revendication 6, dans lequel le dispositif de retrait de capuchon (150) n'est pas rotatif par rapport au carter.

8. Procédé d'assemblage d'un auto-injecteur comprenant :
la fourniture d'un ensemble inférieur (300), l'ensemble inférieur comprenant :
un carter inférieur (310) ayant une extrémité proximale et une extrémité distale, et définissant un axe longitudinal ;
et un ensemble de retrait de protection (100), selon l'une des revendications 1 à **5,** agencé à l'extrémité distale du carter inférieur (310), dans lequel l'au moins une ouverture (122) du dispositif de retenue est espacée de l'extrémité distale (160) du dispositif de retrait de capuchon (150) dans une configuration initiale ;
l'insertion d'une seringue (200) dans l'ensemble inférieur, la seringue (200) comprenant :
un corps (210) ayant une extrémité proximale et une extrémité distale
une aiguille (220) agencée à l'extrémité distale du corps (210) ; et
une protection d'aiguille amovible (230) ayant une extrémité proximale (232) comprenant au moins un élément de saisie complémentaire (233) et une extrémité distale (234), la protection d'aiguille (230) étant configurée pour entourer au moins partiellement l'aiguille (220),
dans lequel l'étape d'insertion comprend le déplacement de la seringue (200) vers l'extrémité distale du carter inférieur (310) le long de l'axe longitudinal de sorte que l'au moins un élément de saisie (125) du dispositif de retenue (110) soit espacé longitudinalement de et situé de manière proximale par rapport à l'au moins un élément de saisie complémentaire (233) de la protection d'aiguille (230) ; et
la mise en place d'un ensemble supérieur comprenant un carter supérieur sur l'ensemble inférieur (300).

9. Procédé de la revendication 8, dans lequel l'au moins un élément de saisie (125) du dispositif de retenue (110) est une saillie (125) configurée pour venir en prise avec un bord proximal de la protection d'aiguille (230), et dans lequel l'étape d'insertion de la seringue (200) dans l'ensemble inférieur comprend la poussée de la seringue (200) de manière distale de sorte que l'au moins une saillie (125) soit positionnée de manière proximale et soit espacée longitudinalement du bord proximal de la protection d'aiguille (230).

10. Procédé de la revendication 8 ou 9, comprenant en outre une étape, après l'insertion de la seringue (200) dans l'ensemble inférieur (300), de solliciter la seringue (200) de manière proximale de sorte que l'au moins un élément de saisie (125) vienne en prise avec le bord proximal de la protection d'aiguille.

11. Procédé de la revendication 10, dans lequel l'étape de solliciter la seringue (200) de manière proximale comprend l'insertion d'un dispositif à travers l'au moins une ouverture (162) dans l'extrémité distale (160) du dispositif de retrait de capuchon (150) et l'ouverture (122) dans l'extrémité distale (120) du dispositif de retenue (110) pour solliciter la seringue (200) de manière proximale.

12. Procédé de l'une des revendications 9 à 11, dans lequel l'extrémité proximale du corps de seringue (210) comprend une bride (240) et l'extrémité proximale du carter inférieur (310) comprend une bague (320).

13. Procédé de la revendication 12, dans lequel, après l'insertion de la seringue (200), la bride de seringue (240) vient en butée contre la bague (320).

14. Procédé de la revendication 13, comprenant en outre, après l'insertion de la seringue (200) dans l'ensemble inférieur (300), une étape de solliciter la seringue (200) de manière proximale, et dans lequel, après l'étape de solliciter la seringue (200) de manière proximale, la bride de seringue (240) est espacée longitudinalement de la bague (320) de la protection d'aiguille.

15. Procédé de l'une des revendications 8 à 14, dans lequel, après l'assemblage, l'extrémité distale (120) du dispositif de retenue (110) tombe ou est déplacée d'une autre manière vers l'extrémité distale (160) du dispositif de retrait de capuchon (150), de sorte que l'extrémité distale (120) du dispositif de retenue (110) vienne en butée contre l'extrémité distale (160) du dispositif de retrait de capuchon (150).
